# EUROPEAN PATENT APPLICATION

(11) **EP 2 862 519 A1**
(43) Date of publication of application: **22.04.2015**
(21) Application number: 14179275.4
(22) Date of filing: 31.07.2014
(51) Int. Cl.: A61B 8/12, A61C 9/00, A61B 5/00, A61B 8/00, A61B 8/08

(54) **Dental ultrasonic diagnostic apparatus**

(30) Priority: 16.10.2013 KR 20130123207
(71) Applicant: Samsung Medison Co., Ltd., Gangwon-do 250-870 (KR)
(72) Inventor: Kim, Tae Young, Seoul (KR); Yoon, Jeong Un, Incheon (KR); Kim, Yu Ri, Seoul (KR); Jin, Gil Ju, Seoul (KR)
(74) Representative: Schmid, Wolfgang

(57) **Abstract**

Disclosed herein is a dental ultrasonic diagnostic apparatus. The dental ultrasonic diagnostic apparatus includes a housing (20) located to be close to teeth and gums during insertion of the housing into a mouth of an object, and an acoustic portion (10) provided inside the housing to transmit and receive an ultrasonic wave. The diagnostic apparatus may diagnoses tissue, foreign bodies, innervation, disease condition, lesions, and the like by ultrasonically imaging a gum, a lip, a palate, and a tongue in the mouth.

## Description

### BACKGROUND

### 1. Field

Embodiments of the present invention relate to a dental ultrasonic diagnostic apparatus to generate an ultrasonic image of gums and teeth of an object using ultrasonic waves.

### 2. Description of the Related Art

An ultrasonic diagnostic apparatus is an apparatus which projects ultrasonic waves from a surface of an object toward a target part inside the object and noninvasively obtains an image related to a monolayer of soft tissue or a blood stream using information of ultrasonic signals (ultrasonic echo signals) reflected therefrom.

Such an ultrasonic diagnostic apparatus may be small, cheap, and display a diagnostic image in real time, compared to other image diagnostic devices such as an X-ray diagnostic device, an X-ray CT scanner (computerized tomography scanner), an MRI (magnetic resonance image) device, and a nuclear medicine diagnostic device. In addition, since the ultrasonic diagnostic apparatus does not cause radiation exposure such as X-ray exposure, the ultrasonic diagnostic apparatus may be inherently safe. Accordingly, the ultrasonic diagnostic apparatus is widely utilized for cardiac, abdominal, and urologic diagnosis as well as maternity diagnosis.

The ultrasonic diagnostic apparatus include an ultrasonic probe which transmits ultrasonic waves onto an object and receives ultrasonic echo signals reflected from the object in order to ultrasonically image the interior of the object.

The ultrasonic diagnostic apparatus include a transducer. Here, the transducer may include a piezoelectric layer which mutually converts electric signals and acoustic signals during vibration of a piezoelectric substance, a matching layer which reduces an acoustic impedance difference between the piezoelectric layer and the object such that ultrasonic waves by the piezoelectric layer generated may be maximally transferred to the object, a lens layer which focuses ultrasonic waves moving forward of the piezoelectric layer on a specific point, and an acoustic absorption layer which blocks ultrasonic waves from moving backward of the piezoelectric layer to prevent image distortion.

### SUMMARY

Therefore, it is an aspect of the present invention to provide a dental ultrasonic diagnostic apparatus capable of performing ultrasonic detection by locating the diagnostic apparatus in the vicinity of teeth and gums of an object during insertion of the diagnostic apparatus into a mouth of the object, in order to measure condition of the teeth and the gums.

Additional aspects of the invention will be set forth in part in the description which follows and, in part, will be obvious from the description, or may be learned by practice of the invention.

In accordance with one aspect of the present invention, a dental ultrasonic diagnostic apparatus includes a housing located to be close to teeth and gums during insertion of the housing into a mouth, and an acoustic portion provided inside the housing to transmit and receive an ultrasonic wave.

The dental ultrasonic diagnostic apparatus may further include a guide portion which guides movement of the acoustic portion and the guide portion may have a rail form.

The dental ultrasonic diagnostic apparatus may further include a power portion which provides power such that the acoustic portion moves on the guide portion and the power portion may transversely move or rotate the acoustic portion in a direction perpendicular to the guide portion.

The housing located to be close to the teeth and gums may have a mouthpiece form and be fixed to front and rear surfaces of the teeth and gums.

The housing may have a stick form and be fixed to a front or rear surface of the teeth and gums, the housing may have a band form and be fixed to a front or rear surface of the teeth and gums, and the housing may be fixed to at least one tooth.

The dental ultrasonic diagnostic apparatus may further include a communication portion which is provided inside the housing, receives a control signal from a control signal input unit outside the housing, and transmits the received control signal to a power portion.

The acoustic portion of the dental ultrasonic diagnostic apparatus may convert an ultrasonic signal reflected from an object into an image signal and transmit the converted image signal to a main body outside the housing.

The dental ultrasonic diagnostic apparatus may further include a gradient adjustment portion in which a gradient value of the acoustic portion measured by a sensor is displayed on a display device of a main body and a power portion may adjust the gradient of the acoustic portion such that the acoustic portion has a gradient value equal to a target gradient value.

### BRIEF DESCRIPTION OF THE DRAWINGS

These and/or other aspects of the invention will become apparent and more readily appreciated from the following description of the embodiments, taken in conjunction with the accompanying drawings of which:
FIG. 1A is a projection view illustrating an ultrasonic diagnostic apparatus according to an embodiment of the present invention;
FIG. 1B is a side view when the ultrasonic diagnostic apparatus is located within a mouth of an object according to an embodiment of the present invention;
FIG. 1C is a front view when the ultrasonic diagnostic apparatus is located within the mouth of the object according to an embodiment of the present invention;
FIG. 2 is a cross-sectional view illustrating an acoustic portion according to an embodiment of the present invention;
FIG. 3A is a projection view illustrating an ultrasonic diagnostic apparatus in which an acoustic portion transversely moves according to an embodiment of the present invention;
FIG. 3B is a view conceptually illustrating transverse movement of the acoustic portion according to an embodiment of the present invention;
FIG. 4A is a projection view illustrating transversely movable power portions attached to side surfaces of the acoustic portion according to an embodiment of the present invention;
FIG. 4B is a side projection view illustrating the transversely movable power portion according to an embodiment of the present invention;
FIG. 4C is a front projection view illustrating the transversely movable power portion according to an embodiment of the present invention;
FIG. 5A is a projection view illustrating transversely movable and vertically rotatable power portions attached to side surfaces of an acoustic portion according to an embodiment of the present invention;
FIG. 5B is a side projection view illustrating the transversely movable and vertically rotatable power portion according to an embodiment of the present invention;
FIG. 5C is a front projection view illustrating the transversely movable and vertically rotatable power portion according to an embodiment of the present invention;
FIG. 5D is a view conceptually illustrating transverse movement and vertical rotation of the acoustic portion according to an embodiment of the present invention;
FIGS. 6A and 6B are perspective views illustrating an external appearance of a housing having a mouthpiece form according to an embodiment of the present invention;
FIG. 6C is a side view when an ultrasonic diagnostic apparatus having a mouthpiece form is located within the mouth of the object according to an embodiment of the present invention;
FIG. 7 is a projection view illustrating an ultrasonic diagnostic apparatus which has a mouthpiece form and is provided with fixed acoustic portions according to an embodiment of the present invention;
FIG. 8 is a projection view illustrating an ultrasonic diagnostic apparatus which has a mouthpiece form and is provided with transversely movable acoustic portions according to an embodiment of the present invention;
FIG. 9 is a projection view illustrating an ultrasonic diagnostic apparatus which has a mouthpiece form and is provided with transversely movable and vertically rotatable acoustic portions according to an embodiment of the present invention;
FIG. 10 is a projection view illustrating an ultrasonic diagnostic apparatus which has a mouthpiece form and is fixed to at least one tooth according to an embodiment of the present invention;
FIG. 11A is a perspective view illustrating a housing having a stick form according to an embodiment of the present invention;
FIG. 11B is a projection view illustrating an ultrasonic diagnostic apparatus which has a stick form and is provided with a fixed acoustic portion according to an embodiment of the present invention;
FIG. 11C is a side view when the ultrasonic diagnostic apparatus having a stick form is located within the mouth of the object according to an embodiment of the present invention;
FIG. 12A is a perspective view illustrating a housing having a band form according to an embodiment of the present invention;
FIG. 12B is a projection view illustrating an ultrasonic diagnostic apparatus which has a band form and is provided with a fixed acoustic portion according to an embodiment of the present invention;
FIG. 12C is a front view when the ultrasonic diagnostic apparatus having a band form is located within the mouth of the object according to an embodiment of the present invention;
FIG. 13 is a block diagram illustrating control of an ultrasonic diagnostic apparatus via a control signal input unit according to an embodiment of the present invention;
FIG. 14 is a block diagram illustrating a process in which an ultrasonic signal reflected from an acoustic portion are received to be converted into an image signal and transmit the converted image signal to a main body according to an embodiment of the present invention; and
FIG. 15 is a flowchart illustrating a method of adjusting a gradient of an acoustic portion by measuring a gradient value of the acoustic portion and setting the gradient value to a gradient value required for the object or to an initial gradient value by push of a reset button according to an embodiment of the present invention.

### DETAILED DESCRIPTION

Reference will now be made in detail to the embodiments of the present invention, examples of which are illustrated in the accompanying drawings, wherein like reference numerals refer to like elements throughout. However, when it is determined that detailed description of known relevant technologies or configurations may unnecessarily obscure the gist of the present invention in the description thereof, the detailed description thereof will be omitted.

Terms used herein are terms defined in consideration of functions of the present invention, and these may vary with the intention or practice of a user or an operator. Therefore, unless otherwise defined, all terms used herein have the same meaning as commonly understood by one of ordinary skill in the art. It will be further understood that terms, such as those defined in commonly used dictionaries, should be interpreted as having a meaning that is consistent with their meaning in the context of the relevant art and the present disclosure, and will not be interpreted in an idealized or overly formal sense unless expressly so defined herein.

Particular features, structures, or characteristics described in connection with the embodiment are included in at least one embodiment of the present disclosure and not necessarily in all embodiments. Furthermore, the particular features, structures, or characteristics of any specific embodiment of the present disclosure may be combined in any suitable manner with one or more other embodiments or may be changed by those skilled in the art to which the embodiments pertain. Therefore, it is to be understood that contents associated with such combination or change fall within the spirit and scope of the present disclosure.

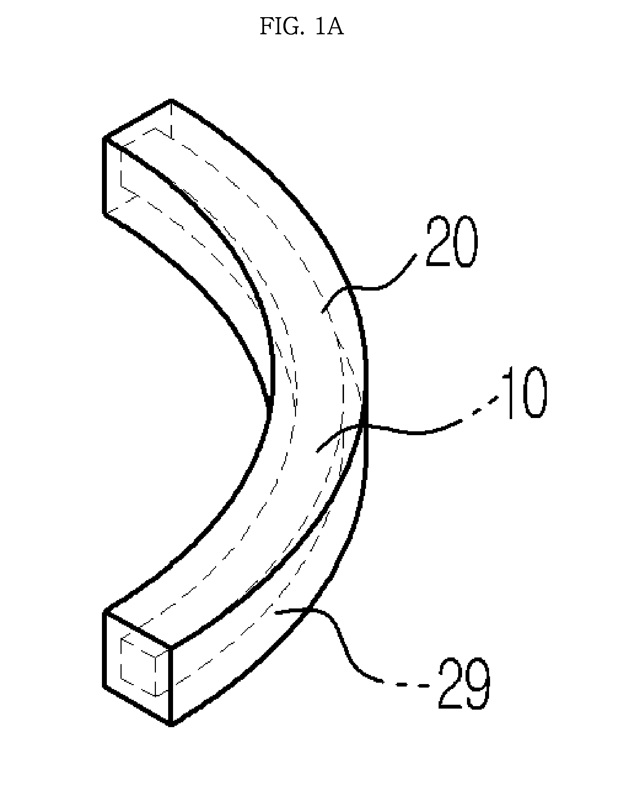

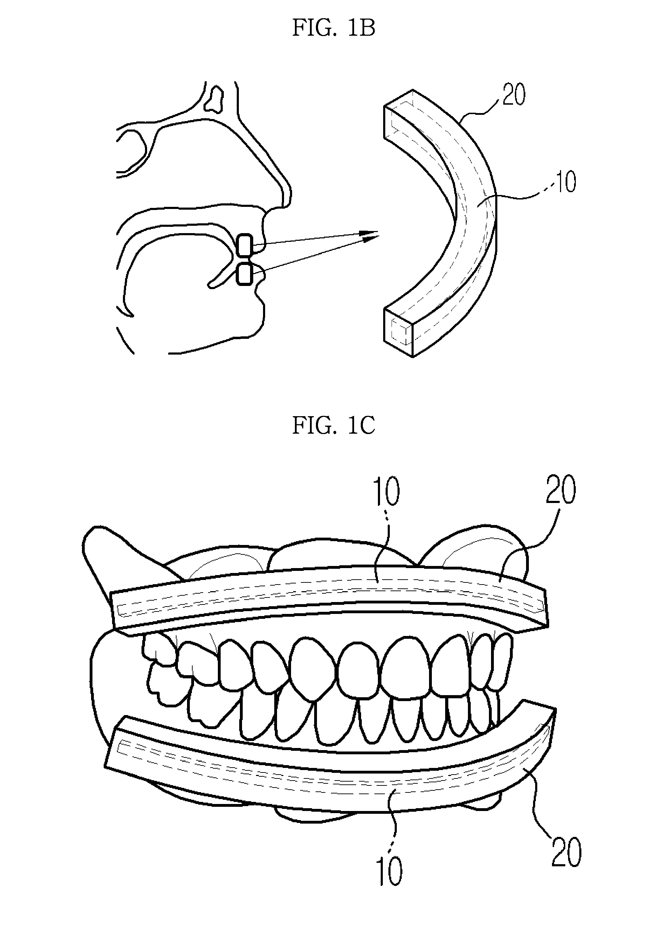

FIG. 1 is a view illustrating a dental ultrasonic diagnostic apparatus according to an embodiment of the present invention. Specifically, FIG. 1A is a projection view illustrating the ultrasonic diagnostic apparatus according to the embodiment. FIG. 1B is a side view when the ultrasonic diagnostic apparatus is located within a mouth of an object according to the embodiment. FIG. 1C is a front view when the ultrasonic diagnostic apparatus is located within the mouth of the object according to the embodiment.

Referring to FIG. 1A, the ultrasonic diagnostic apparatus according to an embodiment of the present invention includes a housing 20 and an acoustic portion 10. The housing 20 has a U-shape and is formed of a length which may cover all teeth of an object. The housing 20 may be made of flexible and elastic material so as to be optimized with respect to the teeth of the object. The acoustic portion 10 is provided inside the housing 20 and has a length equal to that of an inner space of the housing 20 such that the acoustic portion 10 is fixed inside the housing 20. A fluid 29 is provided in an empty space generated when the acoustic portion 10 is fixed inside the housing 20, and allows air inside the housing 20 to be blocked so as to uniformly maintain acoustic impedance. Electric power of the dental ultrasonic diagnostic apparatus according to the embodiment may be supplied by being directly connected to an external main body or by being charged to a battery in the diagnostic apparatus.

Referring to FIGS. 1B and 1C, when the dental ultrasonic diagnostic apparatus according to the embodiment is inserted into a mouth of the object, a part which is closed to teeth and gums may be identified. The ultrasonic diagnostic apparatus may be selectively located at a front or rear surface of the teeth and gums of the object and may be selectively located at an upper or lower portion thereof.

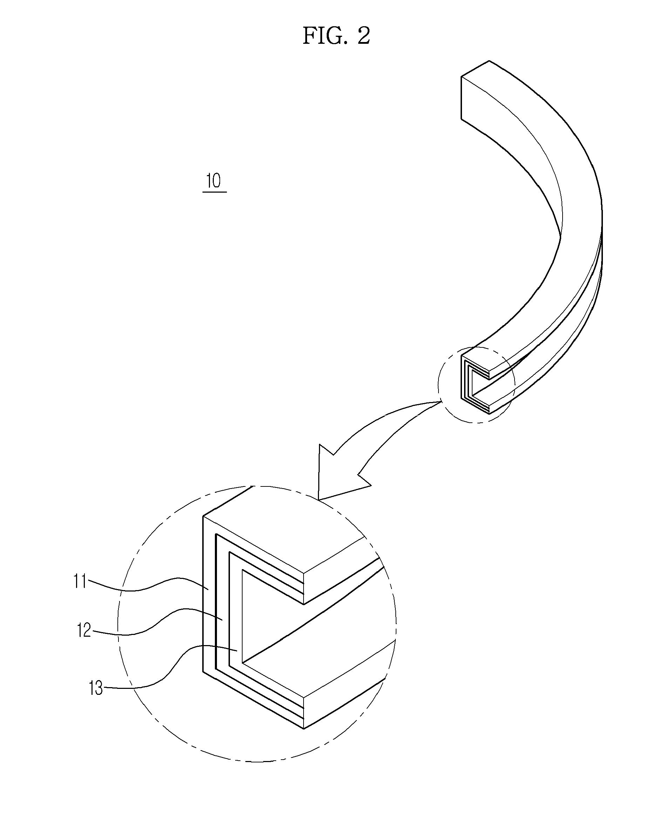

FIG. 2 is a cross-sectional view illustrating the acoustic portion 10 according to an embodiment of the present invention. The acoustic portion 10 includes a matching layer 11, a piezoelectric layer 12, and a backing layer 13. The piezoelectric layer 12 converts electric energy into acoustic energy or converts acoustic energy into electric energy. When the backing layer 13 receives ultrasonic waves reflected from the mouth of the object, the backing layer 13 prevents the ultrasonic waves from being again reflected to the acoustic portion 10. The matching layer 11 functions to improve sensing ability when receiving the reflected ultrasonic waves. As described later, the acoustic portion 10 may convert ultrasonic signals into image signals to transmit the converted image signals to a main body.

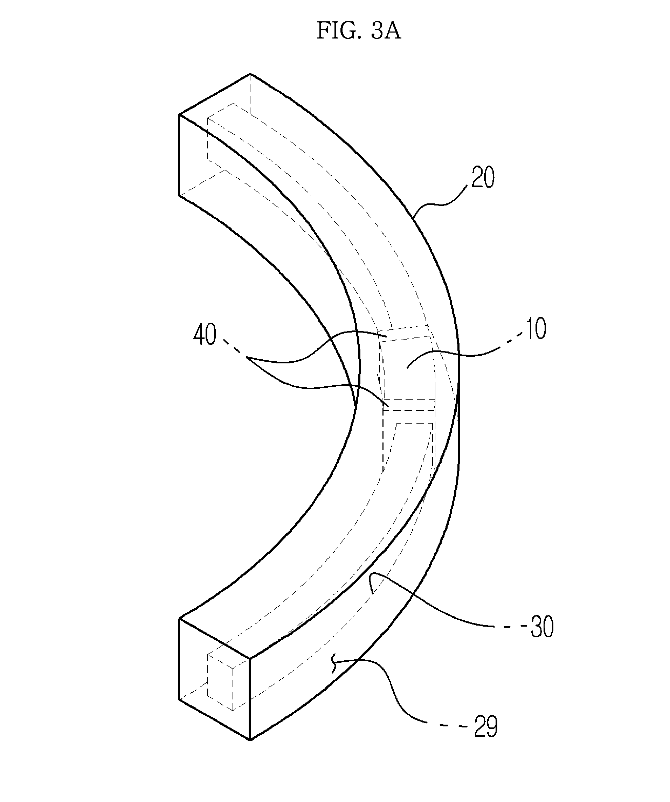

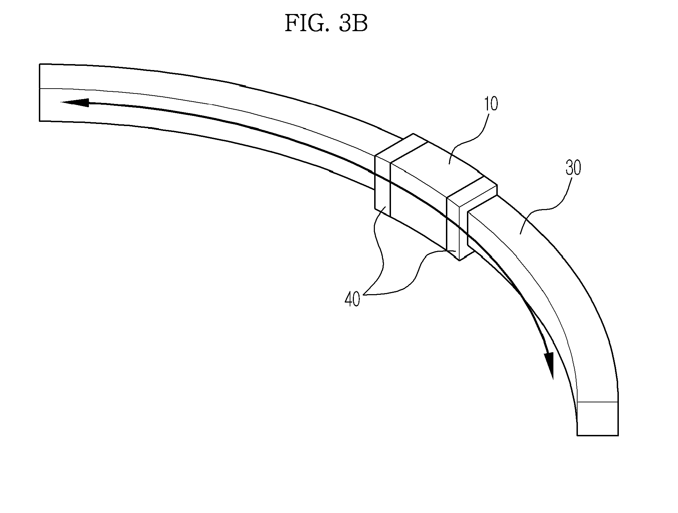

FIG. 3 is a view illustrating an ultrasonic diagnostic apparatus in which an acoustic portion 10 transversely moves along a guide portion 30 according to an embodiment of the present invention. Specifically, FIG. 3A is a projection view illustrating the ultrasonic diagnostic apparatus in which the acoustic portion 10 transversely moves according to the embodiment of the present invention. FIG. 3B is a view conceptually illustrating transverse movement of the acoustic portion 10 according to the embodiment of the present invention.

Referring to FIG. 3A, the ultrasonic diagnostic apparatus according to the embodiment includes an acoustic portion 10, a housing 20, a guide portion 30, power portions 40, and a fluid 29. The housing 20 has a U-shape and is formed of a length which may cover the teeth of the object. The housing 20 may be made of flexible and elastic material so as to be optimized with respect to the teeth of the object. The acoustic portion 10 transmits and receives ultrasonic waves, and has a length equal to the size of one tooth such that the acoustic portion 10 is transversely movable in parallel with the guide portion 30 within the housing 20. The guide portion 30 has a rail form and is located at a front surface within the housing. The guide portion 30 is provided such that the acoustic portion 10 is movable. The power portions 40 are located at both side surfaces of the acoustic portion 10 and provide power such that the acoustic portion 10 is transversely movable. The fluid 29 is provided in an empty space generated when the acoustic portion 10 is fixed inside the housing 20. The fluid 29 allows air inside the housing 20 to be blocked so as to uniformly maintain acoustic impedance or serves to reduce friction during movement of the acoustic portion 10. Referring to FIG. 3B, the acoustic portion 10 transversely moves along the rectangular guide portion 30 by power of the power portions 40.

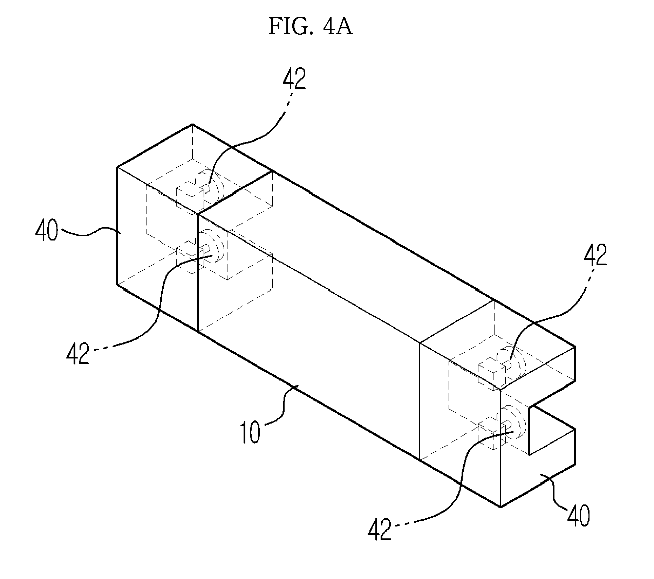

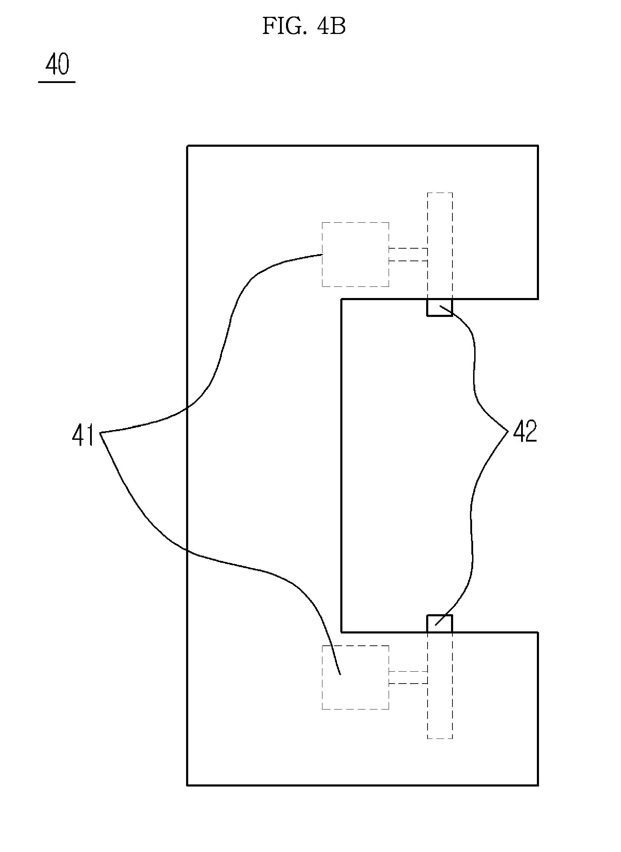

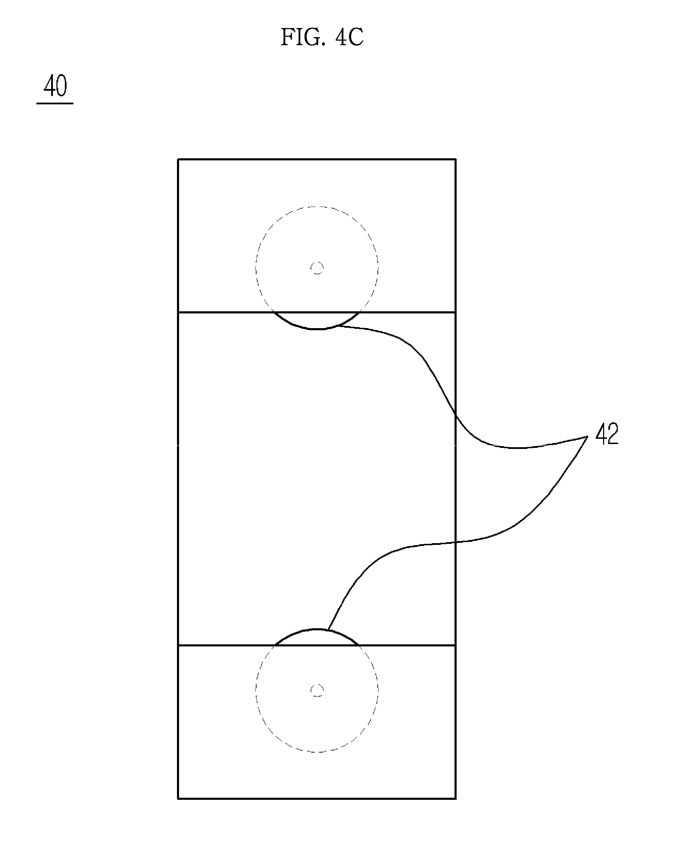

FIG. 4 is a view illustrating transversely movable power portions 40 by which an acoustic portion 10 is transversely movable according to an embodiment of the present invention. Specifically, FIG. 4A is a projection view illustrating the transversely movable power portions 40 attached to side surfaces of the acoustic portion 10 according to the embodiment of the present invention. FIG. 4B is a side projection view illustrating the transversely movable power portion 40 according to the embodiment of the present invention. FIG. 4C is a front projection view illustrating the transversely movable power portion 40 according to the embodiment of the present invention.

Referring to FIGS. 4A to 4C, each of the power portions 40 according to the embodiment includes transversely movable wheels 42 located at upper and lower portions of a groove corresponding to a guide portion 30 and motors 41 providing power to the transversely movable wheels 42. When a control signal is transferred to the power portion 40, the motors 41 operate in response to the control signal and the power of the motors 41 is transferred to the transversely movable wheels 42, thereby allowing both power portions 40 to move. Consequently, the acoustic portion 10 located between both power portions 40 moves along with both power portions 40. Each female/male shape between the guide portion 30, the acoustic portion 10, and the power portions 40 of the dental ultrasonic diagnostic apparatus which is transversely movable may be a rectangular or circular shape.

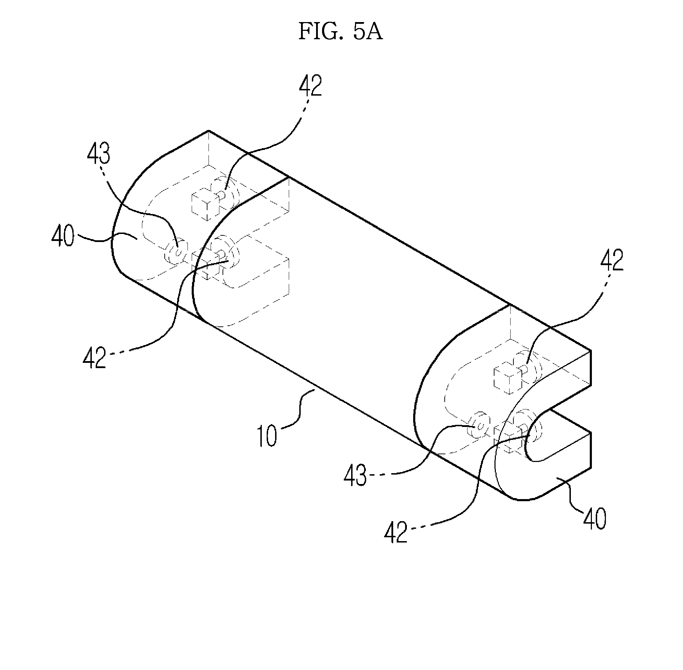

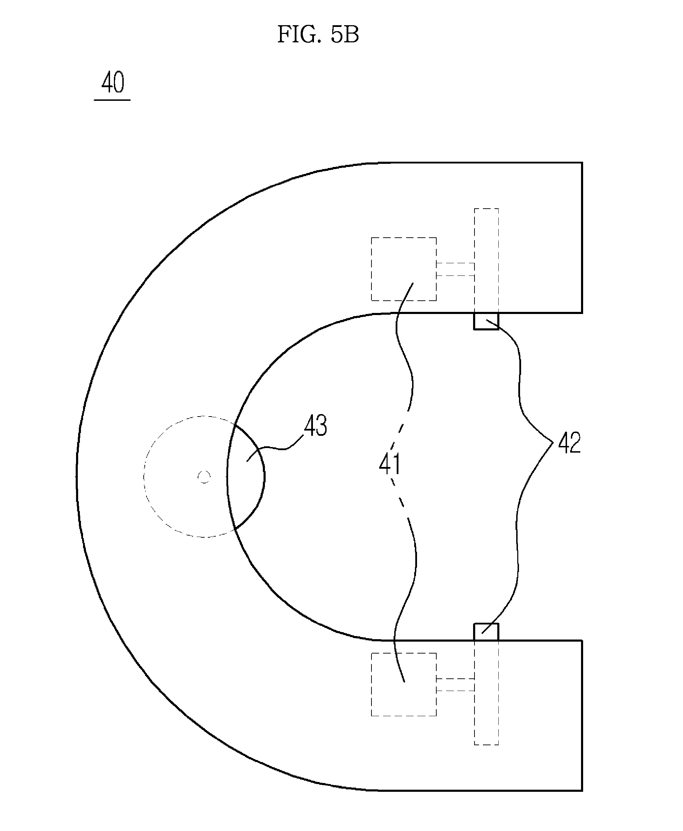

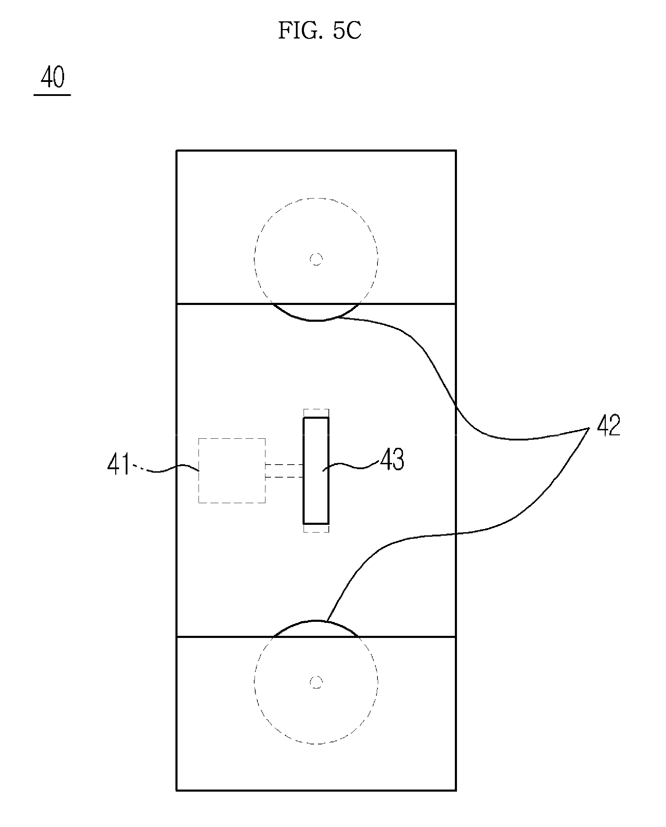

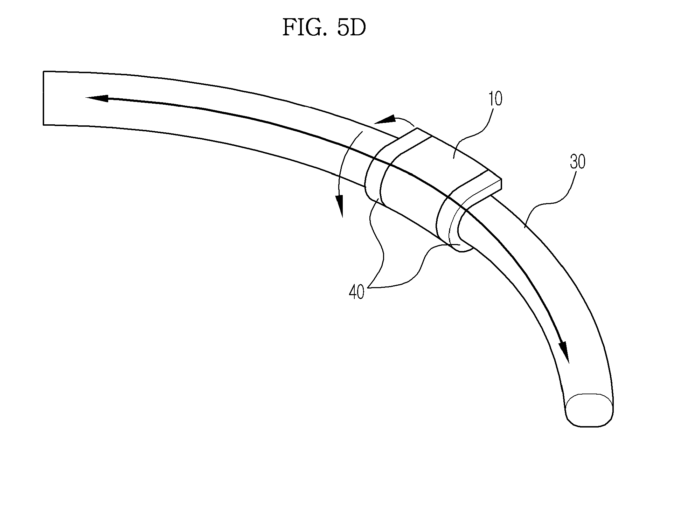

FIG. 5 is a view illustrating power portions 40 by which an acoustic portion 10 is transversely movable and vertically rotatable according to an embodiment of the present invention. Specifically, FIG. 5A is a projection view illustrating the transversely movable and vertically rotatable power portions 40 attached to side surfaces of the acoustic portion 10 according to the embodiment of the present invention. FIG. 5B is a side projection view illustrating the transversely movable and vertically rotatable power portion 40 according to the embodiment of the present invention. FIG. 5C is a front projection view illustrating the transversely movable and vertically rotatable power portion 40 according to the embodiment of the present invention. FIG. 5D is a view conceptually illustrating transverse movement and vertical rotation of the acoustic portion 10 according to the embodiment of the present invention.

Referring to FIGS. 5A to 5C, each of the power portions 40 according to the embodiment includes a vertically rotatable wheel 43 located at a center of a groove corresponding to a guide portion 30 and a motor 41 providing power to the vertically rotatable wheel 43. When a control signal is transferred to the power portion 40, the motor 41 operates in response to the control signal and the power of the motor 41 is transferred to the vertically rotatable wheel 43, thereby allowing both power portions 40 to vertically rotate. Consequently, the acoustic portion 10 located between both power portions 40 rotates along with both power portions 40. Each female/male shape between the guide portion 30, the acoustic portion 10, and the power portions 40 of the dental ultrasonic diagnostic apparatus which is transversely movable may be a circular shape. Referring to FIG. 5D, the acoustic portion 10 may transversely move or vertically rotate on the guide portion 30 by power of the power portions 40.

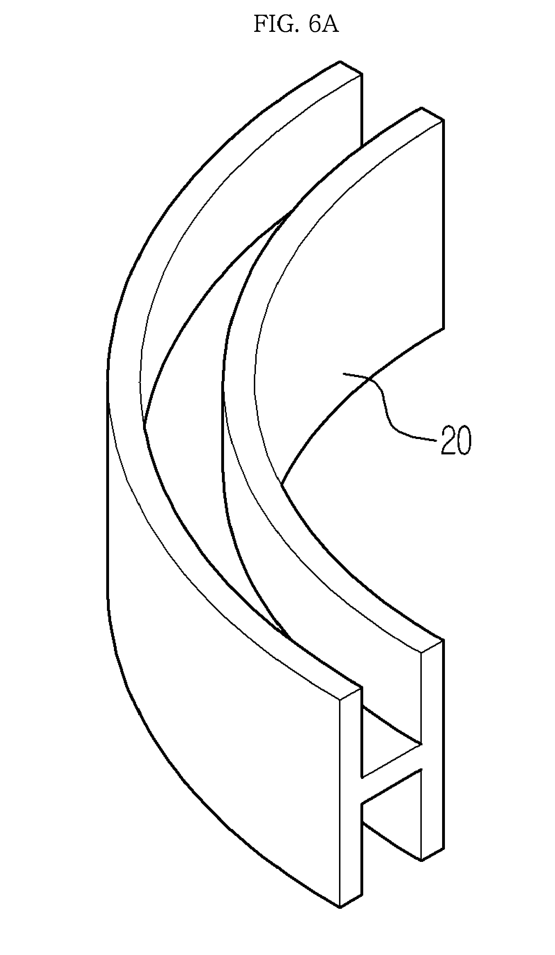

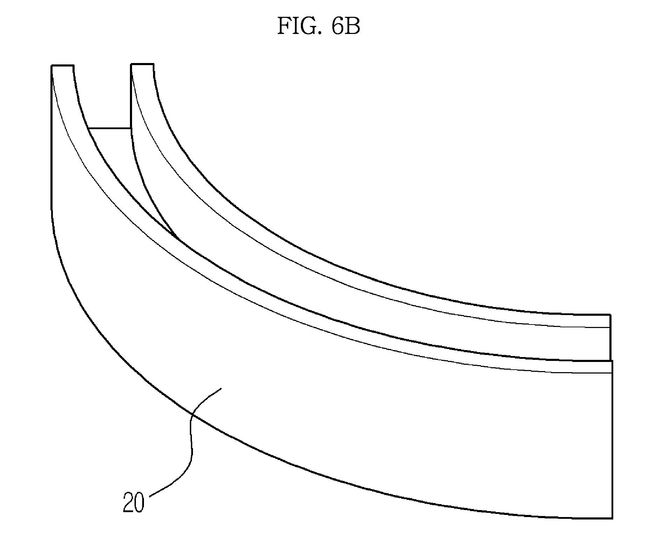

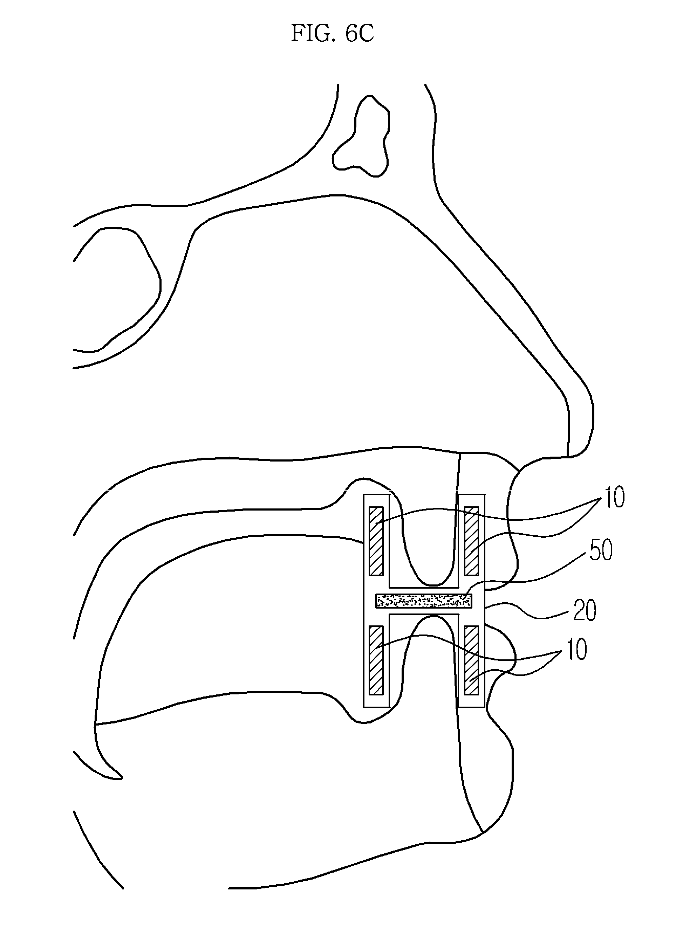

FIG. 6 is a view illustrating a housing 20 having a mouthpiece form according to an embodiment of the present invention. Specifically, FIGS. 6A and 6B are perspective views illustrating an external appearance of the housing 20 having a mouthpiece form according to the embodiment of the present invention. FIG. 6C is a side view when an ultrasonic diagnostic apparatus having a mouthpiece form is located within the mouth of the object according to the embodiment of the present invention.

Referring to FIGS. 6A to 6C, the housing 20 having a mouthpiece form has an H-shape or an N-shape when viewed from the side thereof. The housing 20 is fixed to the teeth and gums by force biting the object in a state of covering the front or rear surface of the teeth and gums of the object when the housing 20 is fixed inside the mouth. Although the housing 20 having a mouthpiece form is simultaneously fixed to upper and lower portions of the teeth and gums of the object, the present invention is not limited thereto. For example, the housing 20 may also be fixed to only one side of the upper and lower portions. The housing 20 may be made of flexible and elastic material so as to be optimized with respect to the teeth of the object.

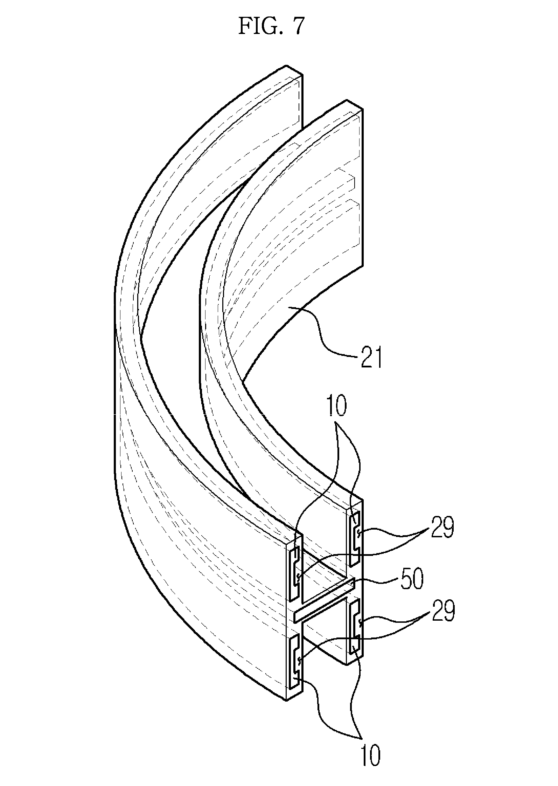

FIG. 7 is a projection view illustrating an ultrasonic diagnostic apparatus which has a mouthpiece form and is provided with fixed acoustic portions 10 according to an embodiment of the present invention. Referring to FIG. 7, a fixed mouthpiece housing 21 has an H-shape when viewed from the side thereof. The acoustic portions 10 are located at side surfaces within the housing 21, and a communication portion 50, which communicates with a control signal input unit outside the diagnostic apparatus, is located at a center of the housing 21. A fluid 29 is provided in an empty space generated when the acoustic portions 10 and the communication portion 50 are located inside the housing 21, and allows acoustic impedance to be uniformly maintained.

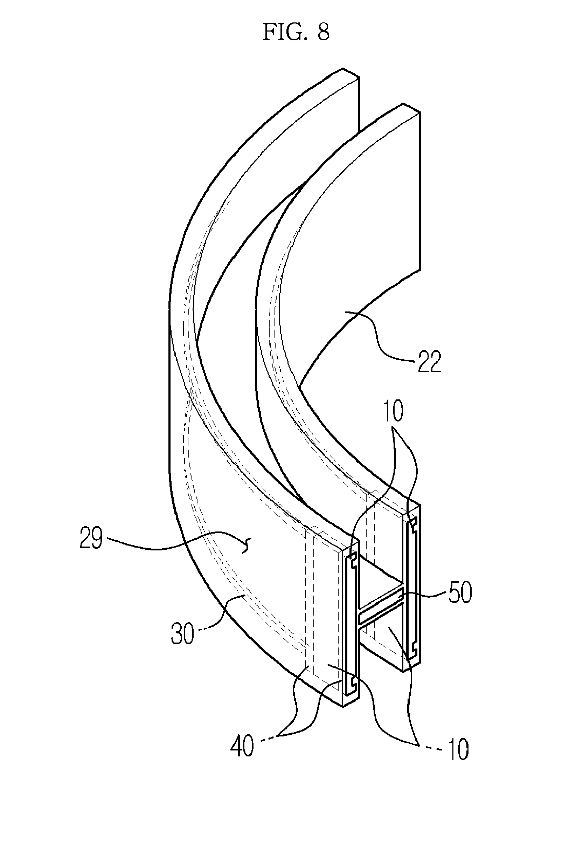

FIG. 8 is a projection view illustrating an ultrasonic diagnostic apparatus which has a mouthpiece form and is provided with transversely movable acoustic portions 10 according to an embodiment of the present invention. Referring to FIG. 8, a transversely movable mouthpiece housing 22 has an H-shape when viewed from the side thereof. The acoustic portions 10 are located at side surfaces within the housing 22, and guide portions 30 having a rail shape are located at upper and lower portions of the side surfaces. Power portions 40 are located at both side surfaces of each acoustic portion 10 to provide power for transverse movement of the acoustic portion 10. A communication portion 50, which communicates with a control signal input unit outside the diagnostic apparatus, is located at a center of the transversely movable housing 22. A fluid 29 is provided in an empty space generated when the acoustic portions 10 and the communication portion 50 are located inside the housing 22, and allows acoustic impedance to be uniformly maintained. The fluid 29 serves to reduce friction during movement of the acoustic portions 10.

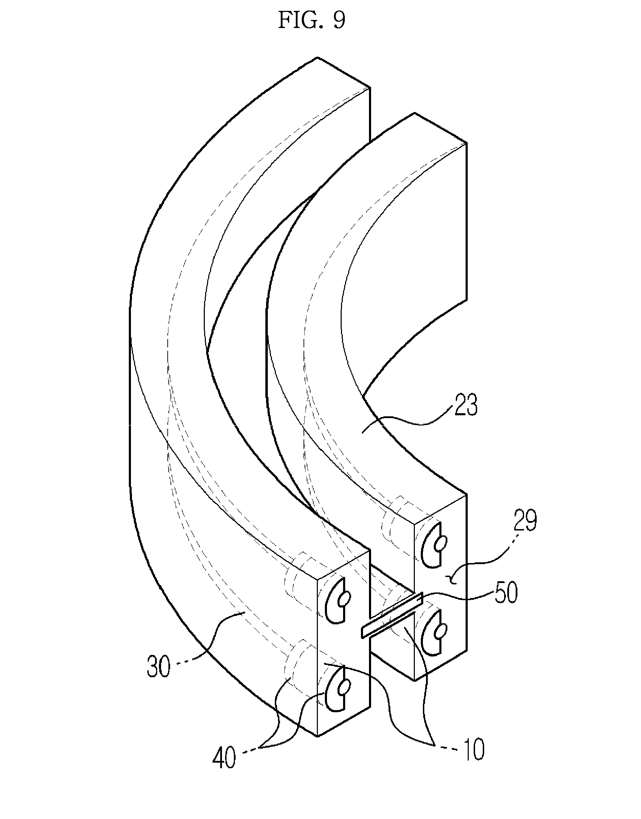

FIG. 9 is a projection view illustrating an ultrasonic diagnostic apparatus which has a mouthpiece form and is provided with transversely movable and vertically rotatable acoustic portions 10 according to an embodiment of the present invention. Referring to FIG. 9, a vertically rotatable mouthpiece housing 23 has an H-shape when viewed from the side thereof. The acoustic portions 10 are located at side surfaces within the housing 23, and guide portions 30 having a rail shape are located at upper and lower portions of the side surfaces. Power portions 40 are located at both side surfaces of each acoustic portion 10 to provide power for vertical rotation and transverse movement of the acoustic portion 10. A communication portion 50, which communicates with a control signal input unit outside the diagnostic apparatus, is located at a center of the vertically rotatable housing 23. A fluid 29 is provided in an empty space generated when the acoustic portions 10 and the communication portion 50 are located inside the housing 23, and allows acoustic impedance to be uniformly maintained. The fluid 29 serves to reduce friction during movement of the acoustic portions 10.

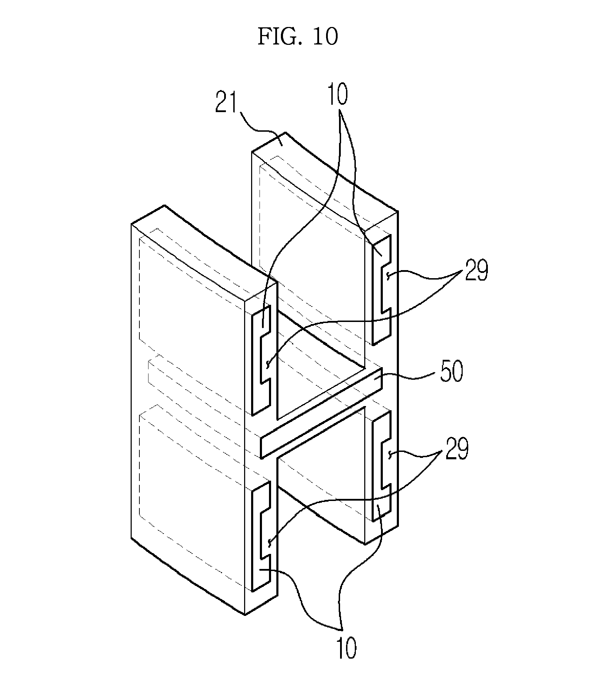

FIG. 10 is a projection view illustrating an ultrasonic diagnostic apparatus which has a mouthpiece form and is fixed to at least one tooth according to an embodiment of the present invention. Referring to FIG. 10, a fixed mouthpiece housing 21 has an H-shape when viewed from the side thereof. Acoustic portions 10 are located at side surfaces within the housing 21, and a communication portion 50, which communicates with a control signal input unit outside the diagnostic apparatus, is located at a center of the housing 21. A fluid 29 is provided in an empty space generated when the acoustic portions 10 and the communication portion 50 are located inside the housing 21, and allows acoustic impedance to be uniformly maintained.

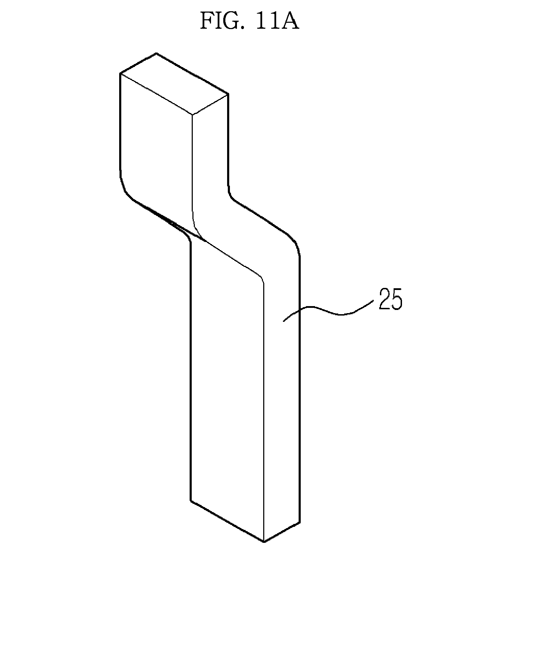

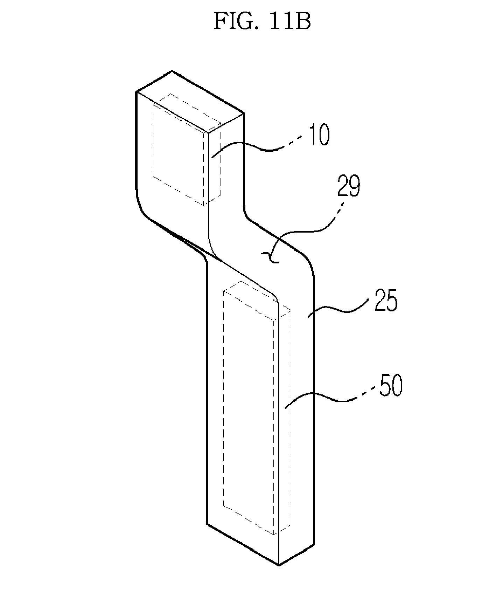

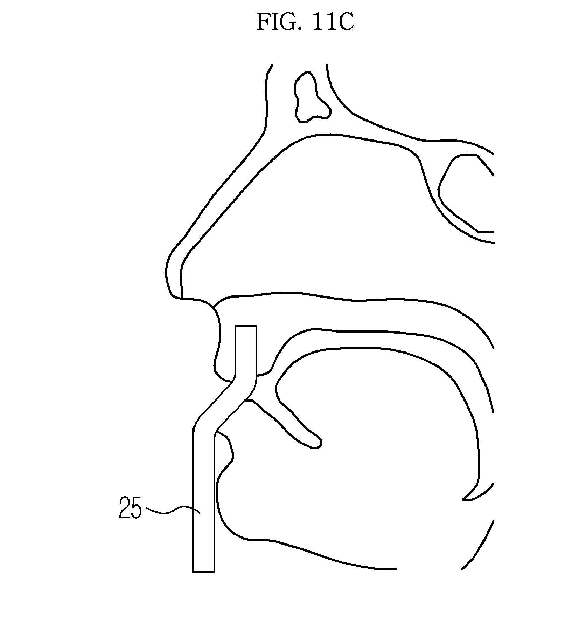

FIG. 11 is a view illustrating a dental ultrasonic diagnostic apparatus having a stick form according to an embodiment of the present invention. Specifically, FIG. 11 A is a perspective view illustrating a housing 25 having a stick form according to the embodiment of the present invention. FIG. 11B is a projection view illustrating the ultrasonic diagnostic apparatus which has a stick form and is provided with a fixed acoustic portion according to the embodiment of the present invention. FIG. 11C is a side view when the ultrasonic diagnostic apparatus having a stick form is located within the mouth of the object according to the embodiment of the present invention.

Referring to FIG. 11B, an upper portion of the stick housing 25 is bent to be inserted into the mouth of the object and an acoustic portion 10 is located inside the housing 25. A communication portion 50 is located inside a lower portion of the stick housing 25 corresponding to a handle. A fluid 29 is provided in an empty space generated when the acoustic portion 10 and the communication portion 50 are located inside the stick housing 25, and allows acoustic impedance to be uniformly maintained.

Referring to FIG. 11C, since the upper portion of the stick housing 25 is bent to be close to a desired part of the object, an ultrasonic image within the mouth may be acquired. Although the stick housing 25 may be located at a front surface of the teeth and gums of the object, the present invention is not limited thereto. For example, the stick housing 25 may also be located at a rear surface of the teeth and gums of the object or at the lower teeth and gums.

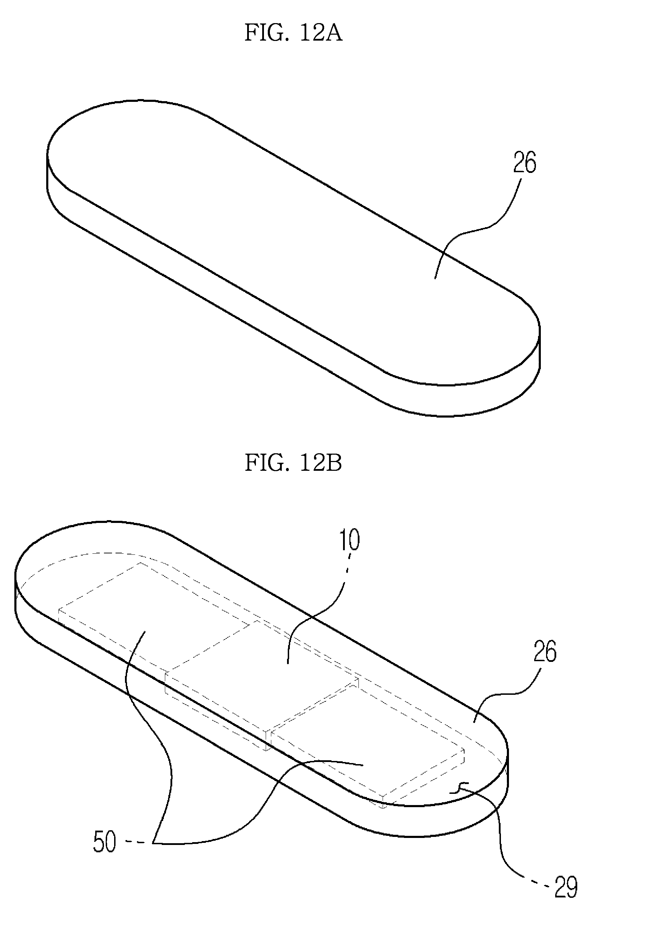

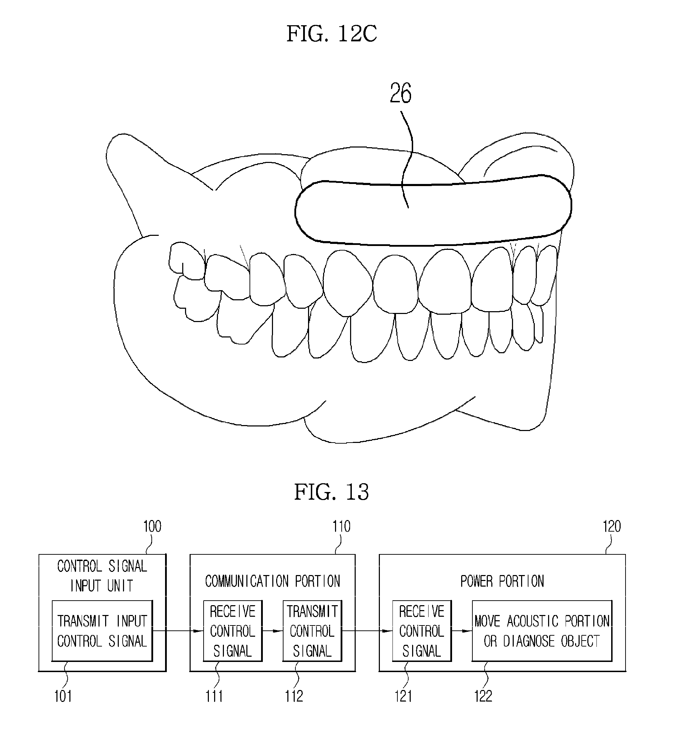

FIG. 12 is a view illustrating a dental ultrasonic diagnostic apparatus having a band form according to an embodiment of the present invention. Specifically, FIG. 12A is a perspective view illustrating a housing 26 having a band form according to the embodiment of the present invention. FIG. 12B is a projection view illustrating the ultrasonic diagnostic apparatus which has a band form and is provided with a fixed acoustic portion 10 according to the embodiment of the present invention. FIG. 12C is a front view when the ultrasonic diagnostic apparatus having a band form is located within the mouth of the object according to the embodiment of the present invention.

Referring to FIG. 12B, the acoustic portion 10 is located inside the band housing 26 and communication portions 50 are located at both side surfaces of the acoustic portion 10. A fluid 29 is provided in an empty space generated when the acoustic portion 10 and the communication portions 50 are located inside the band housing 26, and allows acoustic impedance to be uniformly maintained.

Referring to FIG. 12C, a lower portion of the band housing 26 may come into contact with the teeth and gums of the object and a front surface of the band housing 26 may come into contact with a lip to be fixed thereto. Unlike the form shown in FIG. 12C, the band housing 26 may also be located at a lower portion in the mouth of the object.

FIG. 13 is a block diagram illustrating control of an ultrasonic diagnostic apparatus via a control signal input unit 10 according to an embodiment of the present invention.

The control signal input unit 10 is provided outside the diagnostic apparatus and exchanges signals with a communication portion within the diagnostic apparatus so as to control the diagnostic apparatus. The control signal input unit 10 may include a track ball, a joystick, a resistive touch display, a capacitive touch display, and the like.

Referring to FIG. 13, the control signal input unit 10 transmits an input control signal (operation 101), and a communication portion 110 receives the control signal (operation 111) and transmits the same to a power portion 120 (operation 112). The power portion 120 receives the control signal (operation 121) to move an acoustic portion or diagnose the object (operation 122).

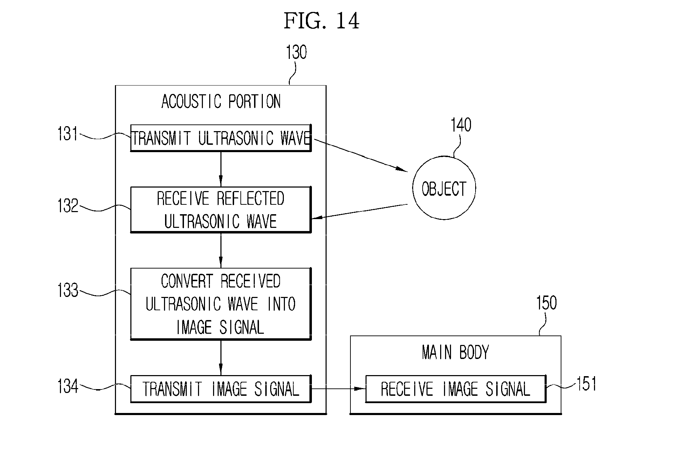

FIG. 14 is a block diagram illustrating a process in which an ultrasonic signal reflected from an acoustic portion 130 are received to be converted into an image signal and transmit the converted image signal to a main body 150 according to an embodiment of the present invention.

Conversion of a received ultrasonic signal into an image signal is performed in main body 150 outside the ultrasonic diagnostic apparatus. However, in accordance with the present embodiment, an ultrasonic signal may be converted into an image signal in the acoustic portion 103 and be transmitted to the main body.

Referring to FIG. 14, an ultrasonic wave is generated by the acoustic portion 130 and is transmitted to an object 140 (operation 131), and then the acoustic portion 130 receives the reflected ultrasonic wave from the object 140 (operation 132). After the received ultrasonic signal is converted into an image signal in the acoustic portion 130 (operation 133), the image signal is transmitted to the main body 150 outside the dental ultrasonic diagnostic apparatus (operation 134). The main body 150 receives the image signal (operation 151) and displays the same on a display device.

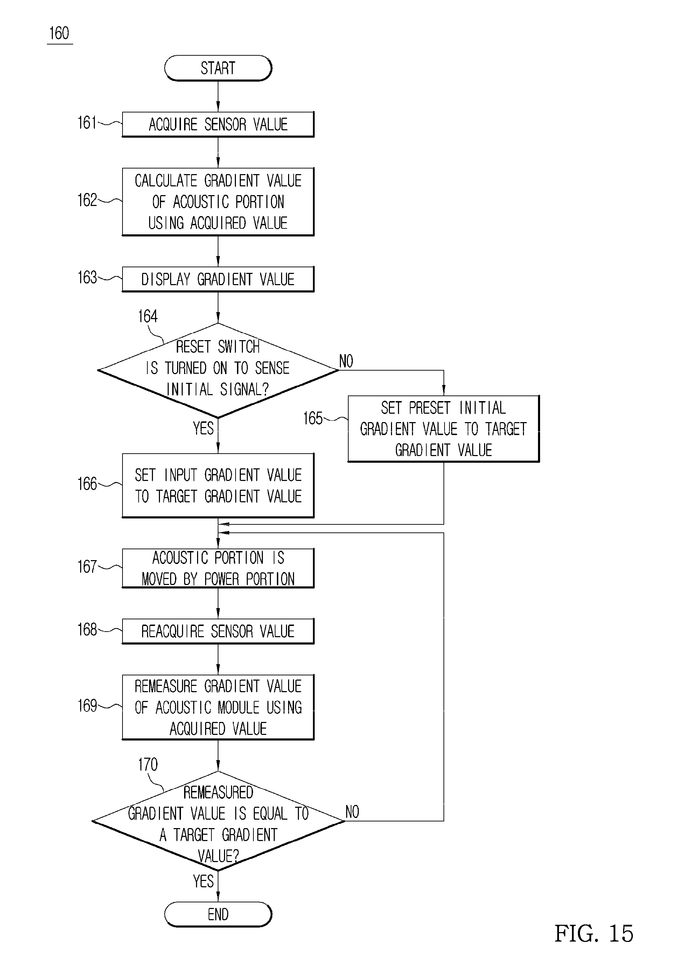

FIG. 15 is a flowchart illustrating a method 160 of adjusting a gradient of an acoustic portion by measuring a gradient value of the acoustic portion and setting the gradient value to a gradient value required for the object or to an initial gradient value by push of a reset button according to an embodiment of the present invention.

Referring to FIG. 15, a value of a sensor measured by the sensor (operation 161), and a gradient value of the acoustic portion is calculated using the acquired value (operation 162). The calculated gradient value is stored and displayed on an external display portion (operation 163). In this case, when a desired gradient value of an object is input, the input gradient value is set to a target gradient value (operation 166). When a reset switch is pushed by the object, an initial signal is generated (operation 164) and a preset initial gradient value is set to a target gradient value (operation 165). An acoustic portion is moved by a power portion (operation 167) such that the gradient value of the acoustic portion is a target gradient value, and the sensor reacquires a value (operation 168) and remeasures a gradient value (operation 169). If the remeasured gradient value is not equal to a target gradient value, the power portion again moves the acoustic portion. Then, it is compared whether or not the gradient value of the acoustic portion is equal to a target gradient value. Here, when the reacquired gradient value is equal to a target gradient value, the power portion stops (operation 170).

The sensor to measure the gradient may be a gyro sensor, an acceleration sensor, or the like.

As is apparent from the above description, in accordance with an ultrasonic diagnostic apparatus according to an embodiment of the present invention, a housing is located such that the diagnostic apparatus is inserted into a mouth to be is close to teeth and gums of an object, ultrasonic waves are generated through an acoustic portion provided within the housing, and the diagnostic apparatus receives the ultrasonic waves reflected from the teeth and gums of the object, enabling tissue such as a gum, a lip, a palate, and a tongue in the mouth, foreign bodies, innervation, disease condition, lesions, and the like to be imaged for diagnosis thereof.

In addition, a 2D image is acquired by transversely moving the acoustic portion along a guide portion and a 3D image is acquired by rotating the acoustic portion in a direction perpendicular to the guide portion, thereby enabling an overall state within the mouth of the object to be imaged. On the contrary, in order to image only a specific section in the mouth required for diagnosis, a desired minimum part of the object may be imaged using the housing fixed to at least one tooth. The ultrasonic diagnostic apparatus may be used to be optimized with respect to the condition of the object by manufacturing the housing in the form of a mouthpiece, a stick, or a band.

In addition, it may be possible to convert ultrasonic signals reflected from the acoustic portion into image signals to transmit the converted image signals to a main body present outside the ultrasonic diagnostic apparatus, and the ultrasonic diagnostic apparatus may be controlled through a communication portion by a control signal input unit present outside the ultrasonic diagnostic apparatus. It may be possible to adjust a gradient of the acoustic portion by measuring a gradient value thereof.

Although a few embodiments of the present invention have been shown and described, it would be appreciated by those skilled in the art that changes may be made in these embodiments without departing from the principles and spirit of the invention, the scope of which is defined in the claims and their equivalents.

## Claims

1. A dental ultrasonic diagnostic apparatus comprising:
a housing located to be close to teeth and gums during insertion of the housing into a mouth; and
an acoustic portion provided inside the housing to transmit and receive an ultrasonic wave.

2. The dental ultrasonic diagnostic apparatus according to claim 1, further comprising a guide portion which guides movement of the acoustic portion.

3. The dental ultrasonic diagnostic apparatus according to claim 2, wherein the guide portion has a rail form.

4. The dental ultrasonic diagnostic apparatus according to claim 2, further comprising a power portion which provides power such that the acoustic portion moves on the guide portion.

5. The dental ultrasonic diagnostic apparatus according to claim 4, wherein the power portion rotates the acoustic portion in a direction perpendicular to the guide portion.

6. The dental ultrasonic diagnostic apparatus according to claim 1, wherein the housing has a mouthpiece form and is fixed to front and rear surfaces of the teeth and gums.

7. The dental ultrasonic diagnostic apparatus according to claim 1, wherein the housing has a stick form and is fixed to a front or rear surface of the teeth and gums.

8. The dental ultrasonic diagnostic apparatus according to claim 1, wherein the housing has a band form and is fixed to a front or rear surface of the

9. The dental ultrasonic diagnostic apparatus according to claim 1, wherein the housing is fixed to at least one tooth.

10. The dental ultrasonic diagnostic apparatus according to claim 1, further comprising a communication portion which is provided inside the housing, receives a control signal from a control signal input unit outside the housing, and transmits the received control signal to a power portion.

11. The dental ultrasonic diagnostic apparatus according to claim 1, wherein the acoustic portion converts an ultrasonic signal reflected from an object into an image signal and transmits the converted image signal to a main body outside the housing.

12. The dental ultrasonic diagnostic apparatus according to claim 1, further comprising:
a sensor which measures a gradient,
wherein a gradient value of the acoustic portion measured by the sensor is displayed on a display device of a main body and a power portion adjusts the gradient of the acoustic portion such that the acoustic portion has a gradient value equal to a target gradient value.
